# EUROPÄISCHE PATENTSCHRIFT

(11) **EP 0 970 052 B1**
(45) Veröffentlichungstag und Bekanntmachung des Hinweises auf die Patenterteilung: **11.08.2004**
(21) Anmeldenummer: 98912427.6
(22) Anmeldetag: 02.03.1998
(51) Int. Cl.: C07D 213/61, C07D 213/38, C07B 43/04

(54) **VERFAHREN ZUR HERSTELLUNG VON 5-AMINOMETHYL-2-CHLORPYRIDINEN**
METHOD FOR PRODUCING 5-AMINOMETHYL-2-CHLOROPYRIDINES
PROCEDE DE PRODUCTION DE 5-AMINOMETHYL-2-CHLOROPYRIDINES

(30) Priorität: 14.03.1997 DE 19710613
(43) Veröffentlichungstag der Anmeldung: 12.01.2000
(73) Patentinhaber: Bayer CropScience AG, 40789 Monheim (DE)
(72) Erfinder: LANTZSCH, Reinhard, D-42115 Wuppertal (DE); STELZER, Uwe, D-51399 Burscheid (DE)
(86) Internationale Anmeldenummer: PCT/EP1998/001163
(87) Internationale Veröffentlichungsnummer: WO 1998/041504

(56) Entgegenhaltungen:
- EP-A- 0 163 855
- EP-A- 0 260 485
- EP-A- 0 458 109
- EP-A- 0 557 967
- DATABASE WPI Section Ch, Week 9349 Derwent Publications Ltd., London, GB; Class B03, AN 93-392633 XP002070902 & JP 05 294 932 A (KOEI CHEM IND CO LTD)

## Beschreibung

Die vorliegende Erfindung betrifft die Herstellung von 5-Aminomethyl-2-chlorpyridinen durch Umsetzung eines Gemisches aus 5-Chlormethyl-2-chlorpyridin und 5-Dichlormethyl-2-chlorpyridin mit substituierten Aminen. Das Gemisch aus 5-Chlormethyl-2-chlorpyridin und 5-Dichlormethyl-2-chlorpyridin wird dabei durch Chlorierung von 5-Methyl-2-chlorpyridin erhalten.

Die Umsetzung von 5-Chlormethyl-2-chlorpyridin mit Aminen zu substituierten 5-Aminomethyl-2-chlorpyridinen ist bekannt (vgl. EP-A 0 391 205). Es ist dazu allerdings erforderlich das Ausgangsprodukt 5-Chlormethyl-2-chlorpyridin in guter Reinheit herzustellen. Dazu wird 5-Methyl-2-chlorpyridin chloriert. Um die Entstehung von höherchlorierten wie 5-Dichlor- oder 5-Trichlormethylpyridinen zu vermeiden, muß die Chlorierung schon bei gringen Umsätzen abgebrochen werden. Das erhaltene Produkt enthält dann hohe Mengen Ausgangsstoff der abgetrennt und der Halogenierung wieder zugeführt werden muß. Die technische Führung dieser Reaktion ist aufwendig.

Es ist bekannt geworden, daß man 5-Aminomethyl-2-chlorpyridine auch erhält, wenn man 5-Trichlormethyl-2-chlorpyridin unter reduzierenden Bedingungen mit Aminen umsetzt. Dabei entstehen jedoch große Mengen salzhaltigen Abwassers - pro Mol Aminomethylpyridin entstehen 3 Mol Chlorid - das aufwendig entsorgt werden muß. Außerdem sind die Ausbeuten bei diesem Prozeß nicht immer zufriedenstellend (vgl. EP-A 0 609 811 und JP-A 5-294932).

Die Seitenkettenchlorierung von 2-Chlor-methylpyridine ist aus EP-A 0 557 967 bekannt, während die Verwendung von substituierten 5-Aminomethyl-pyridinen zur Herstellung von Insektiziden aus EP-A 0 163 855 hervorgeht.

Es wurde nun gefunden, daß man 5-Aminomethyl-2-chlorpyridine der Formel (I) 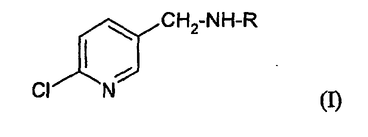 in welcher
- R: für Wasserstoff oder gegebenenfalls substituierten C₁-C₄-Alkyl steht
erhält, wenn man in einer ersten Stufe 5-Methyl-2-chlorpyridin in Form seiner Salze mit anorganischen Säuren gegebenenfalls in Gegenwart eines Verdünnungsmittels bei Temperaturen zwischen 50 und 150°C vorzugsweise in Gegenwart eines Radikalbildners solange chloriert bis der Gehalt an 5-Methyl-2-chlorpyridin < ca. 3 % beträgt und dann gegebenenfalls nach Entfernung des Verdünnungsmittels in einer zweiten Stufe das entstandene Reaktionsgemisch mit Amin der Formel (II)

R-NH₂ (II)

in welcher
- R: für Wasserstoff oder gegebenenfalls substituiertes C₁-C₄-Alkyl steht
unter reduzierenden Bedingungen umsetzt.

Das erfindungsgemäße Verfahren vermeidet die aufwendige selektive Chlorierung von 5-Methyl-2-chlorpyridin bzw. die ebenso aufwendigen Reinigungs- und Trennungsverfahren. Das erfindungsgemäße Verfahren vermeidet die Entstehung großer Mengen salzhaltiger Abwässer.

Es konnte nicht erwartet werden, daß die Umsetzung des Reaktionsgemisches aus 5-Chlormethyl und 5-Dichlormethyl-2-chlorpyridin mit Amin das gewünschte 5-Amino-2-chlorpyridin in guten Ausbeuten und guter Reinheit liefert. Völlig überraschend war, daß die Umsetzung unter Einsatz des Gemisches sogar bessere Ausbeuten liefert als bei Einsatz der entsprechenden reinen Trichlormethyl- oder Dichlormethylpyridine.

Außerdem war überraschend, daß unter den reduktiven Bedingungen eine Abspaltung des 2-Chlor-Atoms am Pyridinkern unterbleibt.

In Formel (I) steht R vorzugsweise für Wasserstoff oder C₁-C₄-Alkyl, das gegebenenfalls durch OH, NH₂ oder -NHR substituiert ist.

R steht insbesondere für Methyl, Ethyl, n-, iso-Propyl, n-Butyl, die gegebenenfalls durch OH, NH₂ oder -NH-(C₁-C₄)-Alkyl substituiert sind.

R steht besonders bevorzugt für Ethyl oder n-Propyl die durch OH, NH₂ oder -NHCH₃ substituiert sind.

In der ersten Stufe werden als Salze von 5-Methyl-2-chlorpyridin bevorzugt Hydrochlorid oder Sulfat eingesetzt. Besonders bevorzugt ist das Hydrochlorid.

Die Chlorierung erfolgt entweder in Gegenwart von Verdünnungsmitteln oder ohne Verdünnungsmitteln in der Schmelze. Als Verdünnungsmittel seien genannt chlorierte Kohlenwasserstoffe wie z.B. Tetrachlorkohlenstoff aber auch Acetonitril oder Wasser.

Die Chlorierung wird bei ca. 50 - 150°C durchgeführt, bevorzugt bei 70 - 120°C.

Die Chlorierung kann vorzugsweise in Gegenwart eines Radikalbildners wie Azoisobutyronitril oder eines Peroxids wie Benzoylperoxid durchgeführt werden.

Der Verlauf der Chlorierung wird laufend z.B. gaschromatographisch verfolgt. Die Chlorierung wird dann abgebrochen, wenn der Gehalt an 5-Methyl-2-chlorpyridin unter ca. 3 % gesunken ist.

Zu diesem Zeitpunkt ist ein Gemisch aus ca. 1 bis 95 % 5-Chlormethyl-2-chlorpyridin und ca. 5 - 99 % 5-Dichlormethyl-2-chlorpyridin entstanden. Dieses Gemisch wird entweder als solches oder nach Abtrennen des bei der Chlorierung eingesetzten Verdünnungsmittels in der 2. Stufe eingesetzt.

In der zweiten Stufe werden ca. 2 bis 30 Äquivalente Amin der Formel II pro Äquivalent umzusetzender Pyridinverbindung eingesetzt.

Bevorzugt werden 3 bis 15 Äquivalente Amin eingesetzt.

Die zweite Stufe der erfindungsgemäßen Reaktion kann in dem in der ersten Stufe eingesetzten Verdünnungsmittel oder nach Abtrennung derselben in einem anderen Verdünnungsmittel durchgeführt werden. Als Verdünnungsmittel seien genannt:
inerte organische Verdünnungsmittel oder Wasser.

### Insbesondere seien genannt:

Wasser, Alkohole, Kohlenwasserstoffe, halogenierte Kohlenwasserstoffe, Ether oder Gemische dieser Lösungsmittel insbesondere mit Wasser.

### Als Alkohole seien genannt:

Methanol, Ethanol, Isopropanol.

### Als Kohlenwasserstoffe seien genannt:

Toluol, Xylol.

### Als halogenierte Kohlenwasserstoffe seien genannt:

Chlorbenzol, Dichlorbenzol.

### Als Ether seien genannt:

Diethylether, Diisopropylether, Methyl-t-butylether, TAME (t-Amyl-methylether)

Besonders bevorzugt sind Gemische von Wasser und Alkoholen.

Die zweite Stufe der Reaktion wird unter reduzierenden Bedingungen durchgeführt. Dazu dient das Arbeiten unter Wasserstoff in Gegenwart von Raney-Nickel oder Raney-Cobalt. Es wird ein Wasserstoffdruck von 1 bis 30 bar, vorzugsweise 1 bis 15 bar eingesetzt.

Die Reaktionstemperatur liegt bei 20 bis 50°C, bevorzugt bei Umgebungstemperatur Die Reaktion wird solange durchgeführt bis kein Wasserstoff mehr aufgenommen wird.

Die Aufarbeitung des bei der 2. Stufe erhaltenen Reaktionsgemisches erfolgt üblicherweise z.B. durch Abfiltrieren des Katalysators, Abdestillieren des Lösungsmittels und der Amine, destillative Reinigung des erhaltenen Rückstands.

Die folgenden Beispiele erläutern den Gegenstand der Erfindung ohne sie in irgend einer Weise einzuschränken.

### Beispiel 1

### Chlorierung von 2-Chlor-5-methylpypridin (CMP) zur Herstellung eines Gemisches aus 2-Chlor-5-chlormethyl/dichlormethyl-pyridin (CCMP/DCMP)

94,8 g (0,6 Mol) 2-Chlor-5-methyl-pyridin-Hydrochlorid werden in 500 ml Tetrachlorkohlenstoff vorgelegt und zum Sieden erhitzt. Dann werden innerhalb von 5,5 Stunden 62,5 g (0,88 Mol) Chlor eingeleitet und simultan 1,8 g Azo-bis-isobuttersäurenitril in 300 ml Tetrachlorkohlenstoff zugetropft. Man erhält ein klares Reaktionsgemisch.

Nach Abkühlen wird mit Wasser versetzt, mit Natronlauge alkalisch gestellt, die organische Phase abgetrennt, einmal mit Wasser gewaschen, getrocknet und eingeengt

Man erhält 103,6 g Chlorierungsgemisch, das lt. gaschromatographischer Analyse zu ca. 45 % aus CCMP und zu 55 % aus DCMP besteht. Der CMP-Gehalt ist <1%.

### Beispiel 2

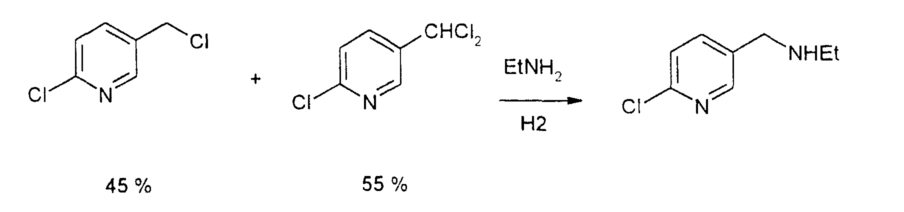

In einem 0,3 1-Autoklaven werden 128,6 g (2 Mol) 70 %ige wäßrige Ethylaminlösung vorgelegt, 5 g Raney-Nickel hinzugefügt und der Autoklav mit Wasserstoff gespült.

Dann werden bei 35°C 30 g Chlorierungsgemisch (45 % CCMP, 55 % DCCP) in 40 ml Ethanol gelöst zugepumpt, während der Wasserstoffdruck bei 9,5 bar gehalten wird. Nach 3 Stunden wird kein weiterer Wasserstoff aufgenommen, es wird nach Abkühlen entspannt, der Katalysator abfiltriert, mit Natronlauge versetzt und Ethylamin + Ethanol abdestilliert.

Die Wasserphase wird noch dreimal mit Toluol extrahiert, die organischen Phasen vereinigt und destillativ aufgearbeitet.

Nach Abdestillieren des Toluols erhält man 26,6 g eines braunen Öls, das durch Destillation gereinigt wird.
Ausbeute: 24,6 g 2-Chlor-5-ethylaminomethylpyridin (86,3 % d.Th.)

### Beispiel 3

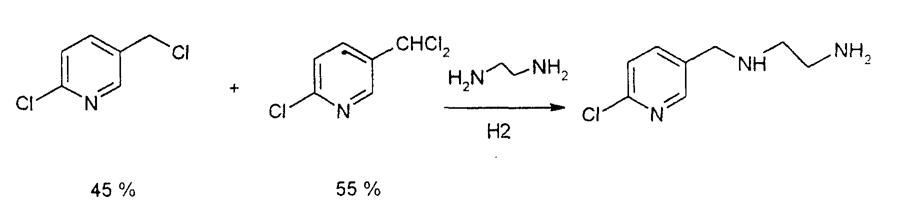

In einem 2 1-Autoklaven werden 436,8 g (7,28 Mol) Ethylendiamin, 239 g Ethanol und 10 g Raney-Nickel vorgelegt und der Autoklav mit Wasserstoff gespült.

Dann werden bei 35°C 102,2 g "Chloriergemisch" (45 % CCMP, 55 % DCCP) in 156 g Ethanol bei 35°C zugepumpt, während der Wasserstoffdruck zwischen 3 und 9 bar gehalten wird. Nach beendeter Wasserstoffaufnahme wird nach Abkühlen auf Raumtemperatur entspannt, der Katalysator abfiltriert, und das Filtrat im Wasserstrahlvakuum bei 40°C eingeengt.

Man erhält 161,6 g klares braunes Öl, das nach HPLC-Analyse mit internem Standard zu 49,8 % aus 2-Chlor-5-aminoethyl-aminomethyl-pyridin besteht, entsprechend einer Ausbeute von 76,0 % d. Th.

### Vergleichsbeispiel A

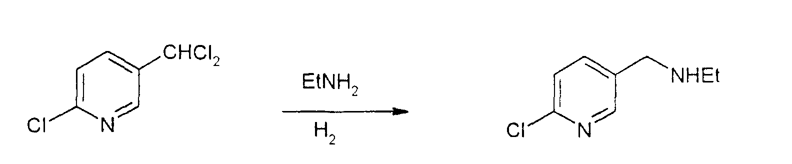

In einem 0,3 1-Autoklaven werden 128,6 g (2,0 Mol) 70 %ige Ethylaminlösung vorgelegt, 5 g Raney-Nickel hinzugefügt und der Autoklav mit Wasserstoff gespült.

Dann werden bei 35°C 49,1 g (0,25 Mol) 5-Dichlormethyl-2-chlor-pyridin gelöst in 70 ml Ethanol zugepumpt, während der Wasserstoffdruck bei 9,5 bar gehalten wird Wenn kein Wasserstoff mehr aufgenommen wird, wird nach Abkühlen auf Raumtemperatur entspannt, der Katalysator abfiltriert, und überschüssiges Ethylamin und Ethanol abdestilliert.

Die Wasserphase wird dreimal mit Toluol extrahiert, die organischen Phasen vereinigt, getrocknet und destillativ aufgearbeitet.

Nach Abdestillieren des Toluols erhält man 34,9g eines hellroten Öls, das im Wasserstrahlvakuum destilliert wird.

Man erhält 21,7 g 2-CWor-5-ethylaminomethylpyridin (50,9 % d.Th.) vom Siedepunkt 115-117°C/5 mbar.

### Vergleichsbeispiel B

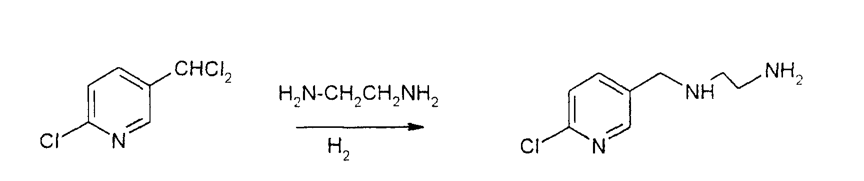

In einem 100 ml-Autoklav werden 42 g 0,7 Mol Ethylendiamin, 23 g Ethanol und 2,5 g Raney-Nickel vorgelegt und der Autoklav mit Wasserstoff gespült. Dann werden bei 35°C 9,8 g (0,05 Mol) Dichlormethyl-chlorpyridin in 10 ml Ethanol gelöst zugepumpt, während der Wasserstoffdruck bei 3 - 9 bar gehalten wird. Nach beendeter Wasserstoffaufnahme wird nach Abkühlen auf Raumtemperatur entspannt, der Katalysator abfiltriert und das Filtrat eingeengt (Wasserstrahlvakuum, 40°C). Man erhält 12,3 g eines klaren, braunen Öls, das nach HPLC-Analyse mit internem Standard zu 39,8 % aus 2-Chlor-5-aminoethyl-aminomethyl-pyridin besteht, entsprechend einer Ausbeute von 52,7 % d.Th.

## Patentansprüche

1. Verfahren zur Herstellung von 5-Aminomethyl-2-chlorpyridinen der Formel (I) 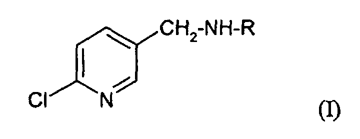 in welcher
R für Wasserstoff oder gegebenenfalls substituiertes C₁-C₄-Alkyl steht,
**dadurch gekennzeichnet, daß** man in einer ersten Stufe 5-Methyl-2-chlorpyridin in Form seiner Salze mit anorganischen Säuren gegebenenfalls in Gegenwart eines Verdünnungsmittels bei Temperaturen zwischen 50 und 150°C vorzugsweise in Gegenwart eines Radikalbildners solange chloriert bis der Gehalt an 5-Methyl-2-chlorpyridin < ca. 3 % beträgt und dann gegebenenfalls nach Entfernung des Verdünnungsmittels in einer zweiten Stufe das entstandene Reaktionsgemisch mit Amin der Formel (II)
R-NH₂ (II)
in welcher
R für Wasserstoff oder gegebenenfalls substituiertes C₁-C₄-Alkyl steht
unter reduzierenden Bedingungen umsetzt.

2. Verfahren gemäß Anspruch 1, wobei Verbindungen der Formel (I) eingesetzt werden, in welcher
R für Wasserstoff oder C₁-C₄-Alkyl, das gegebenenfalls durch OH, NH₂ oder -NHR substituiert ist, steht.

3. Verfahren gemäß Anspruch 1 oder 2, wobei Verbindungen der Formel (I) eingesetzt werden, in welcher
R Methyl, Ethyl, n-, iso-Propyl, n-Butyl, die gegebenenfalls durch OH, NH₂ oder -NH-(C₁-C₄)-Alkyl substituiert sind, steht.

4. Verfahren gemäß Anspruch 1, 2 oder 3, wobei Verbindungen der Formel (I) eingesetzt werden, in welcher
R für Ethyl oder n-Propyl die durch OH, NH₂ oder -NHCH₃ substituiert sind, steht.

5. Verfahren gemäß Anspruch 1, 2, 3 oder 4, **dadurch gekennzeichnet, dass** die Chlorierung in der ersten Stufe bei Temperaturen von 70 bis 120°C durchgeführt wird.

6. Verfahren gemäß Anspruch 1, 2, 3, 4 oder 5, **dadurch gekennzeichnet, dass** die Chlorierung in Gegenwart eines Radikalbildners wie Azoisobutyronitril oder eines Peroxids wie Benzoylperoxid durchgeführt wird.

7. Verfahren gemäß Anspruch 1, 2, 3, 4, 5 oder 6, **dadurch gekennzeichnet, dass** die zweite Stufe bei Temperaturen von 20 bis 50°C durchgeführt wird.

## Claims

1. Process for the preparation of a 5-aminomethyl-2-chloropyridines of the formula (I) 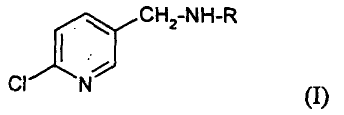 in which
R is hydrogen or optionally substituted C₁-C₄-alkyl,
**characterized in that**, in a first stage, 5-methyl-2-chloropyridine in the form of its salt is chlorinated using inorganic acids optionally in the presence of a diluent at temperatures between 50 and 150°C, preferably in the presence of a free-radical former until the content of 5-methyl-2-chloropyridine is < about 3 %, and then, optionally after the diluent has been removed, in a second stage, the resulting reaction mixture is reacted with amine of the formula (II)
R-NH₂ (II)
in which
R is hydrogen or optionally substituted C₁-C₄-alkyl
under reducing conditions.

2. Process according to Claim 1 utilizing compounds of the formula (I) in which
R is hydrogen or C₁-C₄-alkyl, which is optionally substituted by OH, NH₂ or -NHR.

3. Process according to Claim 1 or 2 utilizing compounds of the formula (I) in which
R is methyl, ethyl, n-, iso-propyl, n-butyl, which are optionally substituted by OH, NH₂ or -NH-(C₁-C₄)-alkyl.

4. Process according to Claim 1, 2 or 3 utilizing compounds of the formula (I) in which
R is ethyl or n-propyl which are substituted by OH, NH₂ or -NHCH₃.

5. Process according to Claim 1, 2, 3 or 4, **characterized in that** the chlorination in the first stage is carried out at temperatures of 70 to 120°C.

6. Process according to Claim 1, 2, 3, 4 or 5, **characterized in that** the chlorination is carried out in the presence of a free-radical former such as azoisobutyronitrile or of a peroxide such as benzoyl peroxide.

7. Process according to Claim 1, 2, 3, 4, 5 or 6, **characterized in that** the second stage is carried out at temperatures of 20 to 50°C.

## Revendications

1. Procédé de production de 5-aminométhyl-2-chloropyridines de la formule (I) 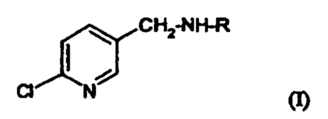 dans laquelle
R représente l'hydrogène ou un alkyle en C₁-C₄ éventuellement substitué,
**caractérisé en ce que** dans une première étape, on chlore de la 5-méthyl-2-chloropyridine sous la forme de sels de celle-ci avec des acides inorganiques, le cas échéant en présence d'un diluant, à des températures comprises entre 50 et 150 °C, de préférence en présence d'un formateur de radicaux, jusqu'à ce que la teneur en 5-méthyl-2-chloropyridine soit environ < 3 % et ensuite, dans une deuxième étape, on fait réagir dans des conditions réductives; le cas échéant après élimination du diluant, le mélange réactionnel obtenu avec une amine de la formule (II)
R-NH₂ (II)
dans laquelle
R représente l'hydrogène ou un alkyle en C₁-C₄ éventuellement substitué.

2. Procédé selon la revendication 1, dans lequel sont mis en oeuvre des composés de la formule (I), dans laquelle
R représente de préférence l'hydrogène ou un alkyle en C₁-C₄, qui est éventuellement substitué par un groupe OH, HN₂ ou -NHR.

3. Procédé selon la revendication 1 ou 2, dans lequel sont mis en oeuvre des composés de la formule (I), dans laquelle
R correspond à un radical méthyle, éthyle, n-, iso-propyle ou n-butyle, qui est éventuellement substitué par un groupe OH, NH₂ ou -NH-(C₁-C₄)-alkyle.

4. Procédé selon la revendication 1, 2 ou 3, dans lequel sont mis en oeuvre des composés de la formule (I), dans laquelle
R représente un radical éthyle ou n-propyle, qui est substitué par un groupe OH, NH₂ ou -NHCH₃.

5. Procédé selon la revendication 1, 2, 3 ou 4, **caractérisé en ce que** la chloration est opérée dans la première étape à des températures comprises entre 70 et 120 °C.

6. Procédé selon la revendication 1, 2, 3, 4 ou 5, **caractérisé en ce que** la chloration est opérée en présence d'un formateur de radicaux tel l'azoisobutyronitrile ou d'un peroxyde tel le peroxyde de benzoyle.

7. Procédé selon la revendication 1, 2, 3, 4, 5 ou 6, **caractérisé en ce que** la deuxième étape est opérée à des températures comprises entre 20 et 50 °C.
